# EUROPEAN PATENT SPECIFICATION

(11) **EP 1 889 827 B1**
(45) Date of publication and mention of the grant of the patent: **25.08.2010**
(21) Application number: 06746829.8
(22) Date of filing: 25.05.2006
(51) Int. Cl.: C07D 295/02

(54) **PROCESS FOR PRODUCING [2-(3,3,5,5-TETRAMETHYLCYCLOHEXYL)PHENYL]PIPERAZINE**
VERFAHREN ZUR HERSTELLUNG VON [2-(3,3,5,5-TETRAMETHYLCYCLOHEXYL)PHENYL]PIPERAZIN
PROCEDE DE PRODUCTION DE [2-(3,3,5,5-TÉTRAMÉTHYLCYCLOHEXYL)PHÉNYL]PIPÉRAZINE

(30) Priority: 25.05.2005 JP 2005151697; 16.12.2005 WO PCT/JP2005/023166
(43) Date of publication of application: 20.02.2008
(73) Proprietor: Eisai R&D Management Co., Ltd., Tokyo 112-8088 (JP)
(72) Inventor: NAGAI, Mitsuo, ISAI Co., Ltd., Tsukuba-shi, Ibaraki 300-2635 (JP); NARA, Kazumasa, EISAI Co., Ltd., Tsukuba-shi, Ibaraki 300-2635 (JP); WAKASUGI, Kazunori, EISAI Co., Ltd., Tsukuba-shi, Ibaraki 300-2635 (JP); NAITO, Toshihiko, EISAI Co., Ltd., Tsukuba-shi, Ibaraki 300-2635 (JP)
(74) Representative: HOFFMANN EITLE
(86) International application number: PCT/JP2006/310450
(87) International publication number: WO 2006/126635

(56) References cited:
- EP-A- 1 698 620
- EP-A- 1 832 580
- EP-A2- 0 748 800
- WO-A-93/11117
- WO-A1-2005/063705
- WO-A1-2006/068058
- FLEMING I. ET AL.: 'Two new oxindole syntheses' JOURNAL OF THE CHEMICAL SOCIETY, PERKIN TRANSACTIONS 1 no. 2, 1986, pages 349 - 359, XP009071954

## Description

### Technical Field

The present invention relates to a process for producing [2-(3,3,5,5-tetramethylcyclohexyl) phenyl] piperazine].

### Background Art

Inflammatory reaction is accompanied by infiltration of leukocytes, typically neutrophils and lymphocytes, into inflammatory sites.
Infiltration of leukocytes is defined as migration of leukocytes such as neutrophils and lymphocytes out of vessels and into the surrounding tissues as a consequence of initiation and activation by cytokines, chemokines, lipids and complement to interact called "rolling" or "tethering" with vascular endothelial cells activated by cytokines such as IL-1 or TNFα, followed by adhesion to the vascular endothelial cells.

As explained below, relationship between leukocyte adhesion or infiltration and various inflammatory diseases and autoimmune diseases was reported. Such reports have raised the possibility that compounds having cell adhesion inhibitory action or cell infiltration inhibitory action may serve as therapeutic or prophylactic agents for such diseases.
(1) Therapeutic or prophylactic agents for inflammatory bowel disease (ulcerative colitis, Crohn's diseases and the like) (see Non-patent documents 1, 2 and 3)
(2) Therapeutic or prophylactic agents for irritable bowel syndrome (see Non-patent document 4)

[Non-patent document 1] Inflammatory Bowel Disease (N. Engl. J. Med., 347: 417-429 (2002))
[Non-patent document 2] Natalizumab for active Crohn's disease (N, Engl. J. Med., 348: 24-32 (2003))
[Non-patent document 3] Granulocyte adsorption therapy in active period of ulcerative colitis (Japanese Journal of Apheresis 18: 117-131 (1999))
[Non-patent document 4] A role for inflammation in irritable bowel syndrome (Gut., 51: i41-i44 (2002))
EP 0 748 800 A2 discloses α₁ adrenoceptor antagonists of formula I 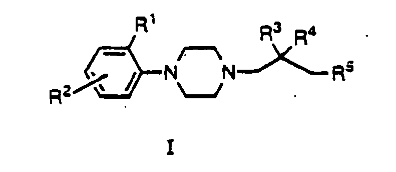 wherein R¹ may be (C₃₋₆) cycloalkyl.

### Disclosure of the Invention

### Problems to be Solved by the Invention

[2-(3,3,5,5-Tetramethylcyclohexyl)phenyl]piperazine compounds are compounds having excellent cell adhesion inhibitory action and cell infiltration inhibitory action, which are useful as therapeutic or prophylactic agents for various inflammatory diseases and autoimmune diseases associated with adhesion and infiltration of leukocytes, such as inflammatory bowel disease (particularly ulcerative colitis or Crohn's disease), irritable bowel syndrome, rheumatoid arthritis, psoriasis, multiple sclerosis, asthma and atopic dermatitis, and the present invention provides a process for the production of [2-(3,3,5,5-tetramethylcyclohexyl)phenyl]piperazine suitable for industrial large-scale production.

### Means for Solving the Problems

As a result of intensive research, the present inventors have discovered a production method of [2-(3,3,5,5-tetramethylcyclohexyl)phenyl]piperazine which is suitable for industrial large-scale production in the following points: (1) purification by column chromatography is not used, (2) explosive intermediates in production are not used, (3) halogenated solvents are not used, and (4) relatively less expensive materials are used. And the present invention was completed on the basis of the discovery.

Specifically, the invention includes the followings:
A process for producing compound (8a): 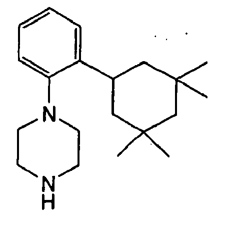
which comprises the reaction of compound (6a): 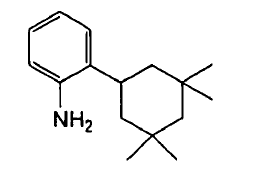 with compound (7a): 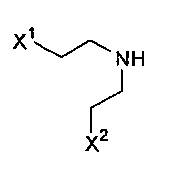 wherein X¹ and X² independently represent a leaving group
in a solvent, wherein the reaction is carried out in the presence of a base.

### Effect of the Invention

The present invention can provide a useful production method of [2-(3,3,5,5-tetramethylcyclohexyl)phenyl]piperazine compounds having cell adhesion inhibitory action and cell infiltration inhibitory action.

### Best Mode for Carrying Out the Invention

The present invention will now be explained in detail.

Throughout the present specification, the structural formulas for the compounds will show only one specific isomer for convenience, but the invention includes all isomers such as geometric isomers, optical isomers, stereoisomers and tautomers implied by the compound structures, as well as their isomer mixtures, and the compounds may therefore be any of the isomers or their mixtures, without being limited to the formulas shown for convenience. The compounds may therefore be in optically active or racemic form, both of which are included without restrictions according to the invention. Polymorphic crystals may also exist, and there may be used any crystal form or a mixture thereof without any restrictions, while the compounds include both anhydrous and hydrated forms.

The definitions of the terms and symbols used throughout the present specification will now be explained, prior to a more detailed description of the invention.

A "halogen" refers to fluorine, chlorine, bromine or iodine.

The term "C1-6 alkyl" refers to a straight-chain or branched C1-6 alkyl group, and as specific examples there may be mentioned methyl, ethyl, 1-propyl (n-propyl), 2-propyl (i-propyl), 2-methyl-1-propyl (i-butyl), 2-methyl-2-propyl (t-butyl), 1-butyl (n-butyl) and 2-butyl (s-butyl).

The term "cyclopropyl-C₁₋₆ alkyl" refers to the above "C₁₋₆ alkyl" bonded to a cyclopropyl group, and as specific examples there may be mentioned cyclopropylmethyl, 2-cyclopropylethyl and 3-cyclopropylpropyl.

The term "C3-8 cycloalkyl" refers to a C3-8 monocyclic saturated aliphatic hydrocarbon group, and as specific examples there may be mentioned cyclopropyl, cyclobutyl, cyclopentyl, cyclohexyl, cycloheptyl and cyclooctyl.

The term "a protecting group for an amino group" is not limited so long as it is a group used for a protecting group for an amino group (Protective Groups in Organic Synthesis, John Wiley & Sons, Inc.), and as specific examples there may be mentioned formyl, pivaloyl, trifluoroacetyl, trichloroacetyl, acetyl, phenylacetyl, benzoyl, N,N-dimethylaminocarbonyl, N-[2-trimethylsilylethoxy]methyl, t-butyloxycarbonyl, methyloxycarbonyl, ethyloxycarbonyl, 2,2,2-trichloroethyloxycarbonyl, 2-trimethylsilyloxycarbonyl, vinyloxycarbonyl, allyloxycarbonyl, benzyloxycarbonyl, p-methoxybenzyloxycarbonyl, p-nitrobenzyloxycarbonyl or benzyl.

### [The meanings of R¹ and R²]

R¹ and R² independently represent hydrogen or a protecting group for an amino group, or R¹ and R² may bond together to form an optionally substituted pyrrole ring, an optionally substituted piperidine ring or an optionally substituted piperazine ring. Preferable examples of R¹ are hydrogen, formyl, pivaloyl, trifluoroacetyl, trichloroacetyl, acetyl, or N,N-dimethylaminocarbonyl, more preferable examples are hydrogen, formyl or pivaloyl, still more preferable examples are formyl or pivaloyl, and the most preferable example is formyl.
Preferable examples of R² are hydrogen, formyl, pivaloyl, trifluoroacetyl, trichloroacetyl, acetyl, or N,N-dimethylaminocarbonyl, more preferable examples are hydrogen, formyl or pivaloyl, still more preferable example is hydrogen.
"Substituent" in "an optionally substituted pyrrole ring" is not limited so long as it is "an optionally substituted pyrrole ring" used as a protecting group for an amino group. Preferable examples of "an optionally substituted pyrrole ring" are a pyrrole ring having 1 to 4 C1-6 alkyl, more preferable examples are a 2,5-dimethylpyrrole ring or a pyrrole ring.
"Substituent" in "an optionally substituted piperidine ring" is not limited so long as it is "an optionally substituted piperidine ring" used as a protecting group for an amino group. Preferable examples of "an optionally substituted piperidine ring" are a non-substituted piperidine ring.
"Substituent" in "an optionally substituted piperazine ring" is not limited so long as it is "an optionally substituted piperazine ring" used as a protecting group for an amino group. Preferable examples of "an optionally substituted piperazine ring" are a non-substituted piperazine ring.

### [The meanings of R¹¹ and R¹²]

R¹¹ and R¹² independently represent hydrogen or a protecting group for an amino group, or R¹¹ and R¹² may bond together to form an optionally substituted pyrrole ring or an optionally substituted piperidine ring.
Preferable examples of R¹¹ are hydrogen, formyl, pivaloyl, trifluoroacetyl, trichloroacetyl, acetyl, or N,N-dimethylaminocarbonyl, more preferable examples are hydrogen, formyl or pivaloyl, still more preferable examples are formyl or pivaloyl, and the most preferable example is formyl.
Preferable examples of R¹² are hydrogen, formyl, pivaloyl, trifluoroacetyl, trichloroacetyl, acetyl, or N,N-dimethylaminocarbonyl, more preferable examples are hydrogen, formyl or pivaloyl, still more preferable example is hydrogen.
"Substituent" in "an optionally substituted pyrrole ring" is not limited so long as it is "an optionally substituted pyrrole ring" used as a protecting group for an amino group. Preferable examples of "an optionally substituted pyrrole ring" are a pyrrole ring having 1 to 4 C1-6 alkyl, more preferable examples are a 2,5-dimethylpyrrole ring or a pyrrole ring.
"Substituent" in "an optionally substituted piperidine ring" is not limited so long as it is "an optionally substituted piperidine ring" used as a protecting group for an amino group. Preferable examples of "an optionally substituted piperidine ring" are a non-substituted piperidine ring.

### [The meanings of R³]

R³ which is described later represents hydrogen, C1-6 alkyl, cyclopropyl-C1-6 alkyl or a protecting group for an amino group, and preferable examples of R³ are hydrogen, C₁₋₆ alkyl, cyclopropyl-C₁₋₆ alkyl, t-butyloxycarbonyl, benzyloxycarbonyl, formyl, pivaloyl, trifluoroacetyl, trichloroacetyl, acetyl, phenylacetyl, benzoyl, p-methoxybenzyl or benzyl, more preferable examples are hydrogen, t-butyloxycarbonyl or benzyloxycarbonyl, still more preferable examples are hydrogen or t-butyloxycarbonyl, and the most preferable example is hydrogen.

### [The meanings of R⁶]

R⁶ which is described later represents C₁₋₆ alkyl or C3-6 cycloalkyl, and preferable examples of R⁶ are n-propyl or cyclopropyl, and more preferable example is cyclopropyl.

### [The meanings of X¹ and X²]

X¹ and X² which are described later independently represent a leaving group, and preferable examples of X¹ and X² are halogen, methanesulfonyloxy or p-toluenesulfonyloxy, more preferable examples are chlorine, bromine, iodine, methanesulfonyloxy or p-toluenesulfonyloxy, still more preferable examples are chlorine or bromine, and the most preferable example is chlorine.

A "salt" as referred to throughout the present specification is not particularly limited so long as it is formed with the compound of the invention, and as examples there may be mentioned inorganic acid salts, organic acid salts and acidic amino acid salts.
As preferable examples of inorganic acid salts there may be mentioned hydrochloride, hydrobromide, sulfate, nitrate and phosphate, and as preferable examples of organic acid salts there may be mentioned oxalate, acetate, succinate, fumarate, maleate, tartarate, citrate, lactate, stearate, benzoate, methanesulfonate, ethanesulfonate, p-toluenesulfonate and benzenesulfonate.
As preferable examples of acidic amino acid salts there may be mentioned aspartate and glutamate.
An acid may form a salt in an appropriate ratio of 0.1 to 5 molecules with 1 molecule of the compound, and preferably forms a salt in an appropriate ration of approximately 1 molecule with 1 molecule of the compound.

The production methods according to the present invention will be explained in detail.
In each formula below, R¹, R², R⁶, X¹ and X² have the same definitions as R¹, R², R⁶, X¹ and X² above, respectively, and T¹ represents leaving group (for example, halogen, methanesulfonyloxy or p-toluenesulfonyloxy, and preferably chlorine).

[Chemical Formula 6]

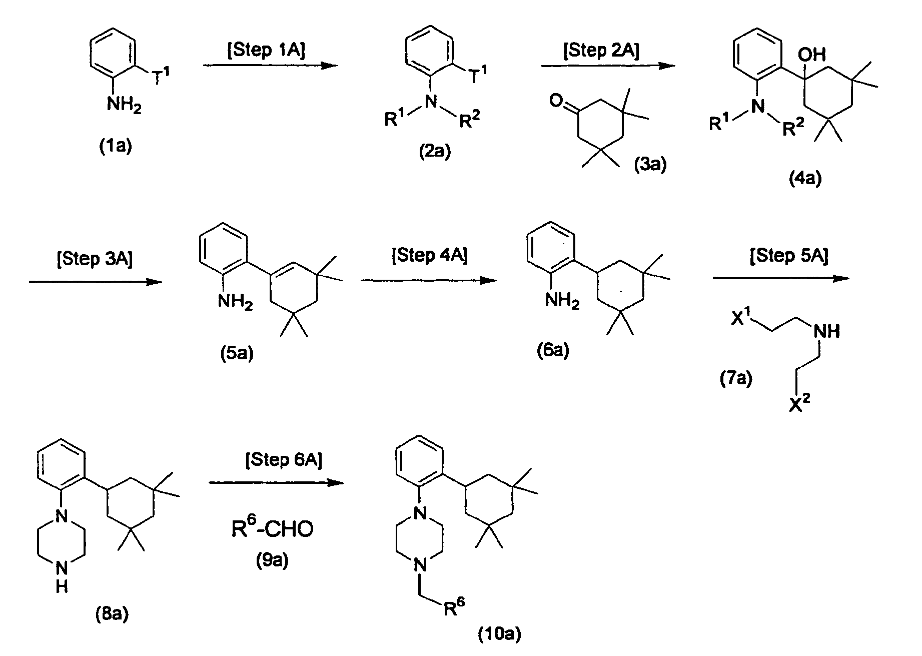

### [Step 1A]

This step is a step of producing compound (2a) by the reaction of compound (1a) with a reagent of introducing a protecting group in a solvent. In this step, the reaction can be carried out in the presence of a base or acid anhydride.

This step can be carried out according to the generally used methods such as those described in Protective Groups in Organic Synthesis, John Wiley & Sons, Inc. and Tetrahedron 1983, 39, 3767-3776. This step can be carried out under the stream or atmosphere of inert gas such as nitrogen or argon.

As compound (1a) may be used publicly known compounds, commercially available compounds or compound easily prepared from commercially available compounds by the methods usually done by those skilled in the art.

The solvent used for this step is not particularly limited so long as it dissolves starting materials to some extent and does not inhibit the reaction, and as examples there may be mentioned ether solvents such as tetrahydrofuran, 1,2-dimethoxyethane, t-butyl methyl ether, cyclopentyl methyl ether, diethyl ether, diisopropyl ether, dibutyl ether and dicyclopentyl ether, aromatic hydrocarbon solvents such as benzene and toluene, aliphatic hydrocarbon solvents such as heptane and hexane, and mixed solvents thereof, and preferable examples are tetrahydrofuran or 1,2-dimethoxyethane.

The reagent of introducing a protecting group is not particularly limited so long as it can introduce a protecting group to nitrogen, and as examples there may be mentioned a reagent for introducing formyl, pivaloyl, trifluoroacetyl, trichloroacetyl, acetyl, phenylacetyl, benzoyl, N,N-dimethylaminocarbonyl, N-[2-trimethylsilylethoxy]methyl, t-butyloxycarbonyl, methyloxycarbonyl, ethyloxycarbonyl, 2,2,2-trichloroethyloxycarbonyl, 2-trimethylsilyloxycarbonyl, vinyloxycarbonyl, allyloxycarbonyl, benzyloxycarbonyl, p-methoxybenzyloxycarbonyl, p-nitrobenzyloxycarbonyl or benzyl, and preferable examples are an acylating reagent or a formylating reagent.

The above acylating reagent refers to pivaloyl chloride, trifluoroacetyl chloride, acetyl chloride, trichloroacetyl chloride, etc., and preferably pivaloyl chloride.
The above formylating reagent refers to combination of acid anhydride such as acetic anhydride and formic acid, or phenyl formate, etc., and preferably combination of acetic anhydride and formic acid.

The above base refers to triethylamine, diisopropylethylamine, pyridine, potassium carbonate, etc., preferably triethylamine.
The above acid anhydride refers to acetic anhydride, propionic anhydride, etc., and preferably acetic anhydride.

The reaction temperature will generally differ depending on the starting materials, the solvent, the other reagents used in the reaction, and it is preferably 0°C to 100°C (internal temperature of the reaction vessel) and more preferably 20°C to 50°C (internal temperature of the reaction vessel).

The reaction time will generally differ depending on the starting materials, the solvent, the other reagents used in the reaction, and the reaction temperature, and stirring at the above reaction temperature for 1 to 5 hours after the addition of the reagents is preferable and stirring for approximately 3 hours is more preferable.

The acylating reagent can be used in an amount of 1- to 2-fold molar amount with respect to compound (1a), and preferably it is used in an amount of 1.05- to 1.25-fold molar.
The formylating reagent can be used in an amount of 1- to 10-fold molar amount with respect to compound (1a), and preferably it is used in an amount of 1.1- to 4-fold molar.

The above base can be used in an amount of 1- to 10-fold molar with respect to compound (1a), and preferably it is used in an amount of 1- to 3-fold molar.
The above acid anhydride can be used in an amount of 1- to 10-fold molar with respect to compound (1a), and preferably it is used in an amount of 2- to 4-fold molar.

### [Step 2A]

This is a step of producing compound (4a) by the reaction of anionized compound (2a), obtainable by the reaction of compound (2a) with a base, with compound (3a) in a solvent. As compound (3a) may be used commercially available compounds.

This step can be carried out according to the generally used methods such as those described in J. Chem. Soc. Perkin. Trans. I, 1986, 349-359, J. Org. Chem., 1984, 49, 2063-2065, Tetrahedron, 1992, 48, 167-176, and Tetrahedron, 1983, 39, 3767-3776.
This step can be carried out under the stream or atmosphere of inert gas such as nitrogen or argon.

The solvent used for this step is not particularly limited so long as it dissolves starting materials to some extent and does not inhibit the reaction, and as examples there may be mentioned ether solvents such as tetrahydrofuran, 1,2-dimethoxyethane, t-butyl methyl ether, cyclopentyl methyl ether, diethyl ether, diisopropyl ether, dibutyl ether and dicyclopentyl ether, aromatic hydrocarbon solvents such as benzene and toluene, aliphatic hydrocarbon solvents such as heptane and hexane, and mixed solvents thereof, and preferable examples are tetrahydrofuran or 1,2-dimethoxyethane.

The above base refers to a butyllithium reagent alone such as n-butyllithium, s-butyllithium and t-butyllithium, or combination of a base selected from Base Group B with the above butyllithium reagent, wherein Base Group B consists of sodium hydride, methyllithium, phenyllithium, potassium hydride, lithium methoxide, sodium methoxide, sodium ethoxide and potassium t-butoxide. Preferable butyllithium reagents include n-butyllithium or t-butyllithium.
Base Group B preferably consists of sodium hydride, methyllithium and phenyllithium, and more preferably consists of sodium hydride and methyllithium.

The reaction temperature will generally differ depending on the starting materials, the solvent, the other reagents used in the reaction, and it is preferably - 100°C to 50°C (internal temperature of the reaction vessel) and more preferably - 80°C to 0°C (internal temperature of the reaction vessel).

The reaction time will generally differ depending on the starting materials, the solvent, the other reagents used in the reaction, and the reaction temperature. Preferably, compound (2a) and a base are stirred at the above reaction temperature for 1 to 3 hours to produce anionized compound (2a). Then, compound (3a) is added to the reaction mixture, followed by stirring at the above temperature for 1 to 20 hours to produce compound (4a). In this case, anionized compound (2a) can be added to the solution containing compound (3a).
More preferably, compound (2a) and a base are stirred at the above reaction temperature for 0.5 to 1 hour, and compound (3a) is added to the reaction mixture, followed by stirring at the above temperature for 1 to 3 hours to produce compound (4a).

The above base can be used in an amount of 1- to 3-fold molar with respect to compound (2a), and preferably it is used in an amount of 1- to 2.2-fold molar.

Compound (3a) can be used in an amount of 0.3- to 3-fold molar with respect to compound (2a), and preferably it is used in an amount of 0.4- to 1-fold molar, and more preferably in an amount of 0.6- to 0.8-fold molar.

### [Step 3A]

This is a step of producing compound (5a) by the reaction of compound (4a) in a solvent in the presence of an acid. This step can be carried out according to the methods generally used for dehydration reaction of alcohol compounds.

This step can be carried out under the stream or atmosphere of inert gas such as nitrogen or argon.

The solvent used for this step is not particularly limited so long as it dissolves starting materials to some extent and does not inhibit the reaction, and as examples there may be mentioned alcohol solvents such as methanol, ethanol, propanol and butanol, ether solvents such as tetrahydrofuran, 1,2-dimethoxyethane, t-butyl methyl ether, cyclopentyl methyl ether, diethyl ether, diisopropyl ether, dibutyl ether and dicyclopentyl ether, aromatic hydrocarbon solvents such as benzene and toluene, aliphatic hydrocarbon solvents such as heptane and hexane, and mixed solvents thereof, and preferable examples are methanol, toluene or heptane.

The above acid refers to pyridinium p-toluenesulfonate, p-toluenesulfonic acid, hydrochloric acid, sulfuric acid, etc., preferably pyridinium p-toluenesulfonate.

The reaction temperature will generally differ depending on the starting materials, the solvent, the other reagents used in the reaction, and it is preferably 30°C to 150°C (internal temperature of the reaction vessel) and more preferably 70°C to 120°C (internal temperature of the reaction vessel).

The reaction time will generally differ depending on the starting materials, the solvent, the other reagents used in the reaction, and the reaction temperature, and stirring at the above reaction temperature for 0.5 to 5 hours after the addition of the reagents is preferable and stirring for approximately 1 hour is more preferable.

The above acid can be used in an amount of 0.01- to 10-fold molar with respect to compound (4a), and preferably it is used in an amount of 0.05- to 1-fold molar and more preferably in an amount of 0.08- to 0.2-fold molar.

### [Step 3A(2)]

In step 3A, a mixture of compound (5a-2) represented by the formula: 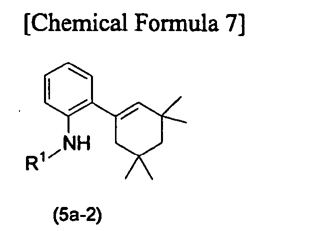 wherein R¹ has the same meaning as R¹ above, and compound (5a) may be obtained. In this case, compound (5a) can be obtained by allowing the mixture of compound (5a-2) and compound (5a) to further react in the presence of a base.

This step can be carried out according to the generally used methods such as those described in Tetrahedron Letters 2004, 45, 9405-9407 and J. Am. Cbem. Soc., 1991, 113, 5085-5086.

This step can be carried out under the stream or atmosphere of inert gas such as nitrogen or argon.

The above base refers to sodium hydroxide, potassium hydroxide, lithium hydroxide, potassium carbonate, sodium carbonate, lithium carbonate, sodium methoxide, potassium methoxide, methylamine, ethylamine, ammonia, etc., and preferably sodium hydroxide or potassium hydroxide.

The solvent used for this step is not particularly limited so long as it dissolves starting materials to some extent and does not inhibit the reaction, and as examples there may be mentioned alcohol solvents such as methanol, ethanol, propanol and butanol, ether solvents such as tetrahydrofuran, 1,2-dimethoxyethane, t-butyl methyl ether, cyclopentyl methyl ether, diethyl ether, diisopropyl ether, dibutyl ether and dicyclopentyl ether, aromatic hydrocarbon solvents such as benzene and toluene, aliphatic hydrocarbon solvents such as heptane and hexane, and mixed solvents thereof, and preferable examples are methanol or ethanol.

The reaction temperature will generally differ depending on the starting materials, the solvent, the other reagents used in the reaction, and it is preferably 20°C to 240°C (internal temperature of the reaction vessel) and more preferably 60°C to 180°C (internal temperature of the reaction vessel).

The reaction time will generally differ depending on the starting materials, the solvent, the other reagents used in the reaction, and the reaction temperature, and stirring at the above reaction temperature for 1 to 30 hours after the addition of the reagents is preferable and stirring for approximately 15 hours is more preferable.

The base can be used in an amount of 0.1- to 100-fold molar with respect to total amount of compound (5a-2) and compound (5a), and preferably it is used in an amount of 0.5- to 2-fold molar.

### [Step 4A]

This is a step of producing compound (6a) by the reaction of compound (5a) in a solvent in the presence of a reduction catalyst under hydrogen atmosphere.
This step can be carried out according to the generally used methods such as those described in Jikken Kagaku Koza, 4th edition (4th series of experimental chemistry), vol. 26, p251-266.

The solvent used for this step is not particularly limited so long as it dissolves starting materials to some extent and does not inhibit the reaction, and as examples there may be mentioned alcohol solvents such as methanol, ethanol, propanol and butanol, ether solvents such as tetrahydrofuran, 1,2-dimethoxyethane, t-butyl methyl ether, cyclopentyl methyl ether, diethyl ether, diisopropyl ether, dibutyl ether and dicyclopentyl ether, aromatic hydrocarbon solvents such as benzene and toluene, aliphatic hydrocarbon solvents such as heptane and hexane, and mixed solvents thereof, and preferable examples are methanol or ethanol.

The above reduction catalyst refers to palladium on carbon, palladium hydroxide, platinum oxide, Raney nickel, and preferably palladium on carbon.

In this step, the reaction can be carried out under hydrogen atmosphere at atmospheric pressure to under pressure (1 to 100 kgf/cm²), and preferably the reaction can be carried out under hydrogen atmosphere at 5 to 15 kgf/cm².

The reaction temperature will generally differ depending on the starting materials, the solvent, the other reagents used in the reaction, and it is preferably 0°C to 60°C (internal temperature of the reaction vessel) and more preferably 15°C to 30°C (internal temperature of the reaction vessel).

The reaction time will generally differ depending on the starting materials, the solvent, the other reagents used in the reaction, and the reaction temperature, and stirring at the above reaction temperature for 5 to 100 hours after the addition of the reagents is preferable and stirring for approximately 15 hours is more preferable.

The above reduction catalyst can be used in an amount of 0.001- to 1-fold molar with respect to compound (5a), and preferably it is used in an amount of 0.01- to 0.06-fold molar and more preferably in an amount of 0.03-fold molar.

### [Step 5A]

This is a step of producing compound (8a) by the reaction of compound (6a) and compound (7a) in a solvent. In this step, the reaction is out in the presence of a base.

This step can be carried out according to the generally used methods such as those described in Chem. Lett., 1998, 8, 2675-2680.

This step can be carried out under the stream or atmosphere of inert gas such as nitrogen or argon. The reaction can be accelerated by combination with microwave.

As compound (7a) may be used publicly know compounds, commercially available compounds or compound easily prepared from commercially available compounds by the methods usually done by those skilled in the art.

The solvent used for this step is not particularly limited so long as it dissolves starting materials to some extent and does not inhibit the reaction, and as examples there may be mentioned aromatic hydrocarbon solvents such as p-cymene, o-dichlorobenzene, diphenyl ether, benzene, toluene and xylene, alcohol solvents such as butanol and ethylene glycol, aliphatic hydrocarbon solvents such as heptane and hexane, ether solvents such as tetrahydrofuran, 1,2-dimethoxyethane, t-butyl methyl ether, cyclopentyl methyl ether, diethyl ether, diisopropyl ether, dibutyl ether and dicyclopentyl ether or mixed solvents thereof, and preferable examples are p-cymene or diphenyl ether.

The above base refers to potassium carbonate, sodium carbonate, triethylamine, etc., and preferably potassium carbonate or sodium carbonate, more preferably potassium carbonate.

The reaction temperature will generally differ depending on the starting materials, the solvent, the other reagents used in the reaction, and it is preferably 50°C to 250°C (internal temperature of the reaction vessel) and more preferably 90°C to 190°C (internal temperature of the reaction vessel).

The reaction time will generally differ depending on the starting materials, the solvent, the other reagents used in the reaction, and the reaction temperature, and stirring at the above reaction temperature for 2 to 15 hours after the addition of the reagents is preferable and stirring for approximately 8 hours is more preferable.

Compound (7a) can be used in an amount of 1- to 3-fold molar with respect to compound (6a), and preferably it is used in an amount of 1- to 2-fold molar, more preferably 1.2- to 2-fold molar.

The above base can be used in an amount of 1- to 10-fold molar with respect to compound (6a), and preferably it is used in an amount of 2- to 5-fold molar, more preferably 3-fold molar.

In step 5A, compound (7a) in situ synthesized by the reaction of diethanolamine with a halogenating reagent or a reagent for introducing a leaving group in a reaction vessel can be used instead of compound (7a) (Synthetic communication, 1998, 28, 1175-1178).
The halogenating reagent refers to thionyl chloride, hydrochloric acid, phosphorus oxychloride, hydrobromic acid, N-bromosuccinimide, N-chlorosuccinimide, etc., and preferably thionyl chloride or hydrochloric acid.
The reagent for introducing a leaving group refers to mesyl chloride, tosyl chloride, etc.

The reaction of diethanolamine with the halogenating reagent or the reagent for introducing a leaving group can be carried out in the presence of a base such as triethylamine. The reaction can be accelerated by combination with microwave.

### [Step 6A]

This is a step of producing compound (10a) by the reaction of compound (8a) and compound(9a) in a solvent in the presence of a reducing agent.
This step can be carried out according to the generally used methods such as those described in Jikken Kagaku Koza, 4th edition (4th series of experimental chemistry), vol. 20, p282-284.

This step can be carried out under stream or atmosphere of inert gas such as nitrogen or argon.

As compound (9a) may be used publicly know compounds, commercially available compounds or compound easily prepared from commercially available compounds by the methods usually done by those skilled in the art.

The solvent used for this step is not particularly limited so long as it dissolves starting materials to some extent and does not inhibit the reaction, and as examples there may be mentioned ether solvents such as tetrahydrofuran, 1,2-dimethoxyethane, t-butyl methyl ether, cyclopentyl methyl ether, diethyl ether, diisopropyl ether, dibutyl ether and dicyclopentyl ether, aromatic hydrocarbon solvents such as benzene and toluene, aliphatic hydrocarbon solvents such as heptane and hexane, and mixed solvents thereof, and preferable examples are tetrahydrofuran or toluene.

The above reducing reagent refers to sodium triacetoxyborohydride, sodium cyanoborohydride, sodium borohydride, etc., and preferably sodium triacetoxyborohydride.

The reaction temperature will generally differ depending on the starting materials, the solvent, the other reagents used in the reaction, and it is preferably 0°C to 50°C (internal temperature of the reaction vessel) and more preferably 10°C to 30°C (internal temperature of the reaction vessel).

The reaction time will generally differ depending on the starting materials, the solvent, the other reagents used in the reaction, and the reaction temperature, and stirring at the above reaction temperature for 1 to 5 hours after the addition of the reagents is preferable and stirring for approximately 1 hour is more preferable.

Compound (9a) can be used in an amount of 1- to 2-fold molar with respect to compound (8a), and preferably it is used in an amount of 1- to 1.5-fold molar and more preferably in an amount of 1- to 1.2-fold molar.

The above reducing reagent can be used in an amount of 0.3- to 2-fold molar with respect to compound (8a), and preferably it is used in an amount of 1.0-to 2.0-fold molar and more preferably in an amount of 1.3- to 1.5-fold molar.

### [Step 7A]

This is a step of producing compound (12a) by the reaction of compound (6a) and compound (11a) in a solvent.

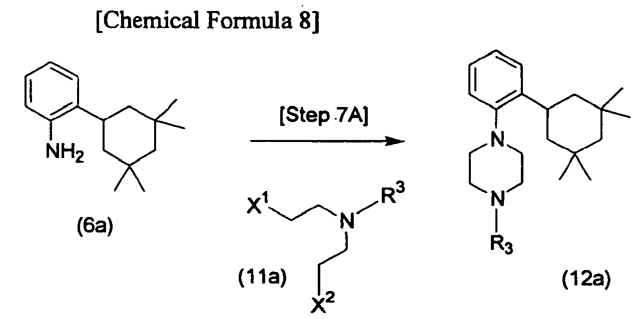

In each formula, R³, X¹ and X² have the same definitions as R³, X¹ and X² above, respectively.
This step can be carried out according to the same methods and conditions as the above step 5A.

As compound (11a) may be used publicly know compounds, commercially available compounds or compound easily prepared from commercially available compounds by the methods usually done by those skilled in the art.
In step 7A, compound (11a) obtainable by the reaction of compound (11a-2) represented by the formula 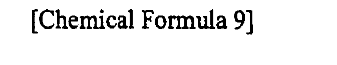 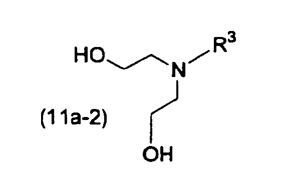 wherein R³ has the same definition as R³ above, with a halogenation reagent or a reagent for introducing a leaving group in a reaction vessel can be used instead of compound (11a). This step can be carried out according to the same methods and conditions as the above step 5A.

The conversion from compound (4a) to compound (6a) can be carried out also by the [Step 3B] to [Step 5B] below.

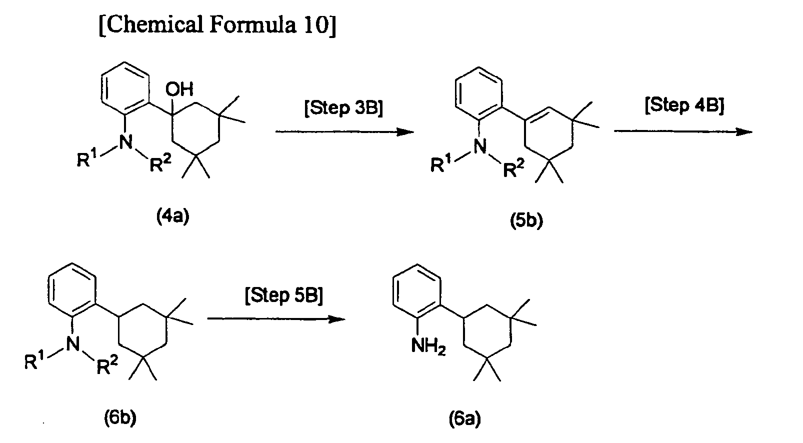

In each formula, R¹ and R² have the same definitions as R¹ and R² above, respectively.

### [Step 3B]

This is a step of producing compound (5b) by the reaction of compound (4a) in a solvent in the presence of an acid.
This step can be carried out according to the same methods and conditions as the above step 3A.

### [Step 4B]

This is a step of producing compound (6b) by the reaction of compound (5b) in a solvent in the presence of a reduction catalyst under hydrogen atmosphere.
This step can be carried out according to the same methods and conditions as the above step 4A.

### [Step 5B]

This is a step of producing compound (6a) by the reaction of compound (6b) in the presence or absence of a solvent, in a base.
This step can be carried out according to the same methods and conditions as the above step 3A(2).

After completion of the reactions of each of the methods and steps described above, the target compound of each step may be recovered from the reaction mixture according to conventional procedures.
For example, when the entire reaction mixture is a liquid, it may be returned to room temperature or cooled on ice if necessary, then allowed to neutralize an acid, an alkali, an oxidizing agent or a reducing agent if necessary, and then water and an organic solvent such as ethyl acetate which is immiscible with water and which does not react with the target compound may be added, and the layer containing the target compound is separated. Next, there may be added a solvent which is immiscible with the resultant layer and which does not react with the target compound, and the layer containing the target compound may be washed and separated. If the layer is an organic layer, it may be dried using a desiccant such as anhydrous magnesium sulfate or anhydrous sodium sulfate, the solvent may be distilled off to recover the target compound. If the layer is an aqueous layer, it may be electrically desalted and then lyophilized to recover the target compound.
When the entire reaction mixture is a liquid, if possible the substances other than the target compound (for example, solvents, reagents, etc.) may be simply distilled off at atmospheric pressure or under reduced pressure to recover the target compound.
When the target compound alone precipitates as a solid, or when the entire reaction mixture is a liquid and the target compound alone precipitates as a solid during the recovery procedure, the target compound may be first filtered by a filtration method and the filtered target compound washed with a suitable organic or inorganic solvent and dried to allow treatment of the mother liquor in the same manner as when the entire reaction mixture is a liquid, in order to recover the target compound.
When only the reagent or catalyst is present in solid form, or when the entire reaction mixture is a liquid and the reagent or catalyst alone precipitates as a solid during the recovery procedure, with the target compound dissolved in the solution, the reagent or catalyst may be first filtered by a filtration method and the filtered reagent or catalyst washed with a suitable organic or inorganic solvent, and then the obtained wash liquids combined with the mother liquor and the obtained mixture treated in the same manner as when the entire reaction mixture is a liquid, in order to recover the target compound.
Particularly when substances other than the target compound in the reaction mixture do not inhibit the reaction of the subsequent step, the reaction mixture may be used directly for the subsequent step without isolation of the target compound.

The purity of the target compound recovered by the method described above may be improved by appropriately employing a recrystallization method, chromatography method or distillation method.
When the recovered target compound is a solid, it will usually be possible to improve the purity of the target compound by recrystallization. For recrystallization, a single solvent or multiple solvents which do not react with the target compound may be used. Specifically, the target compound is first dissolved in the single or multiple solvents which do not react therewith, either at room temperature or with heating. The resulting solution is either cooled on ice or allowed to stand at room temperature to crystallization of the target compound from the mixture.
When the target compound is free form, it can form a salt with an acid or base, and the salt of the target compound can be purified by recrystallization same as described above. After purification, the salt of the target compound can be converted to a free form to produce the target compound with higher purity.
When the recovered target compound is a liquid, the purity of the target compound may be improved by any of various chromatography methods. A weak acid silica gel such as Silica Gel 60 (70-230mesh or 340-400 mesh) by Merck Co. or BW-300 (300 mesh) by Fuji Silysia Chemical Ltd. may be used in most cases. When the target compound is basic and adsorption is too strong on the aforementioned silica gels, Chromatorex-NH silica gel (200-350 mesh) propylamine coated one by Fuji Silysia Chemical Ltd. or the like may be used.
When the target compound is dipolar or must be eluted with a polar solvent such as methanol, NAM-200H or NAM-300H by Nam Research Co. may be used. These silica gels may be used for elution of the target compound with a single solvent or multiple solvents which do not react with the target compound, followed by distilling off of the solvent, to yield the target compound with improved purity.
When the recovered target compound is a liquid, its purity may be improved by a distillation method. For distillation, the target compound is subjected to reduced pressure at room temperature or with heating to distill off the target compound.

Representative examples of production methods of the invention have been described above, but the starting compounds and reagents used for production of the compounds of the invention may also form salts, hydrates or solvates, which will differ depending on the starting materials and solvents used, and are not particularly limited so long as they do not inhibit the reaction. The solvents used will also differ depending on the starting materials and reagents, but of course they are not particularly limited so long as they dissolve the starting materials to some extent and do not inhibit the reaction.
When an intermediate in production of the invention are obtained in the free form, a conventional procedure may be carried out to convert it to a salt or hydrate of the intermediate in production.
When an intermediate in production of the invention is obtained as a salt or hydrate, it may be converted to the free form according to a conventional procedure.

The production methods or intermediates in production of the invention may be performed or produced by the methods described in the following examples. However, these specific examples are merely illustrative and are not intended to restrict the invention in any way, and various modifications may be implemented such as are within the scope of the invention.
The compounds accompanying literatures were prepared according to the literatures.

The term "room temperature" in the Reference Examples and the Examples below ordinarily refers to a temperature between approximately 10°C and 35°C. The percentage values are weight percentages, unless otherwise specified. The other symbols as used herein stand for the followings.
s: singlet
d: doublet
t: triplet
q: quartet
m: multiplet
br: broad
J: coupling constant
Hz: Hertz
CDCl₃: deutero chloroform
NMR: proton nuclear magnetic resonance
Piv: a pivaloyl group (a t-butylcarbonyl group)

### Examples

Silica gel used in the following Examples is silica gel 60 (Merck & Co., Inc) or BW300 (Fuji Silysia Chemical Ltd.) unless otherwise mentioned, and NH silica gel used is Chromatorex-NH silica gel (Fuji Silysia Chemical Ltd.), propylamine-coated one.

### Example 1: 2-(3,3,5,5-tetramethylcyclohexyl)phenylamine oxalate (Reference)

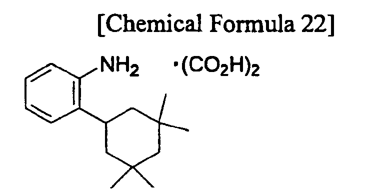

Oxalic acid (121 g) and ethyl acetate (3000 mL) were placed in a 9 L separable flask and the mixture was heated to about 45°C to dissolve oxalic acid.
A mixture of 2-(3,3,5,5-tetramethylcyclohexyl)phenylamine (274.6 g, 1.09 mol) and ethyl acetate (1190 mL) was added dropwise to the solution of oxalic acid with keeping the temperature at around 50°C over about 3.5 hours.
The vessel containing the solution of 2-(3,3,5,5-tetramethylcyclohexyl)phenylamine was washed with ethyl acetate (300 mL), and the mixture was cooled to about 20°C and allowed to stand for about 3 hours.
The crystals were filtered, washed with ethyl acetate (600 mL) and dried under reduced pressure at about 50°C to give the title compound (348.6 g, 99%) as white crystals.

### Example 2 1-[2-(3.3.5.5-tetramethylcyclohexyl)phenyl]piperazine methanesulfonate

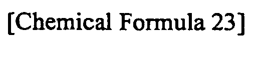

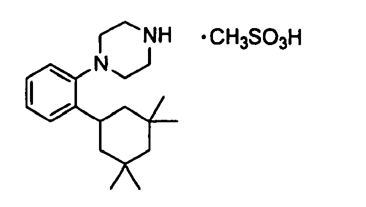

2-(3,3,5,5-Tetramethylcyclohexyl)phenylamine oxalate (338.6 g, 1.05 mol) and toluene (1700 mL) were placed in a 3 L separable flask, and a 1N aqueous solution of KOH (2415 g) was added, followed by stirring for 30 minutes. The mixture was allowed to stand, and the aqueous layer was discarded, and the organic layer was washed with water (1700 mL) and a 5% aqueous solution of sodium chloride in this order.
The organic layer was filtered and insoluble matter was washed with toluene (85 mL), concentrated under reduced pressure to give 2-(3,3,5,5-tetramethylcyclohexyl)phenylamine (244 g, 98.8%) as a pale brown oil.
2-(3,3,5,5-Tetramethylcyclohexyl)phenylamine (264.4 g, 1.098 mol), bis(2-chloroethyl)amine hydrochloride (333 g), N-methyl-2-pyrrolidone (1270 mL) and potassium carbonate (426 g) were placed in a 5L 4-neck round bottom flask.
The mixture was rapidly heated to 170 to 180°C, and the temperature was kept for 7 hours, then the mixture was cooled down to 70°C.
Ethyl acetate (1270 mL) was added, and the reaction mixture was transferred to a 9L separable flask, and water (1270 mL) was added with keeping the temperature at 50°C or higher, and the reaction mixture was heated to about 80°C.
The reaction mixture was filtered using a filter pre-coated with celite, and the residue was washed with ethyl acetate (1270 mL), which was combined with the filtrate and liquid-liquid extraction was performed.
The organic layer was washed twice with water (1270 mL) and concentrated under reduced pressure.
The concentrate was placed in a 5L separable flask, ethyl acetate (4320 mL) was added, and methanesulfonic acid (79.1 g) was added at about 30°C.
After allowing to stand for about 2 hour, the reaction mixture was filtered under reduced pressure, and dried under reduced pressure at about 80°C to give the title compound (233 g, 53.4%) as crude crystals of pale brown solid.

Crude crystals of the title compound (236 g) and 2-propanol (1180 mL) were placed in a 5L separable flask, and the mixture was heated to about 80°C, and ethyl acetate (2360 mL) was added dropwise over about 1 hour.
After allowing to stand at the same temperature for about 0.5 hours, the mixture was cooled down to about 25°C and kept for 1 hour.
The crystals were filtered, washed with a mixed solution of 2-propanol (157 mL) and ethyl acetate (315 mL), and concentrated under reduced pressure at about 80°C to give the title compound (214 g, 90.7%) as white crystals.

### [Example 3] 2-(3,3,5,5-Tetramethylcyclohexyl)phenylamine (Reference)

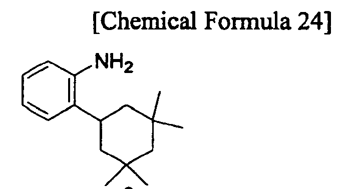

### Example 3-A: N-(2-Bromophenyl)-2.2-dimethylpropionamide

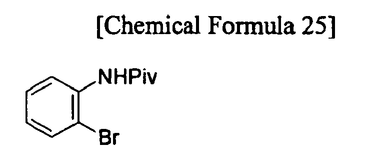

To a solution of 2-bromoaniline (30 g, 174 mmol) in toluene (300 mL) were added triethylamine (35.4 g, 348 mmol) and pivaloyl chloride (21.4 g, 178 mmol) at room temperature, followed by stirring at the same temperature for 2.5 hours. The reaction mixture was washed once with a 1N aqueous solution of hydrochloric acid and twice with a 5% aqueous solution of sodium chloride, dried over magnesium sulfate, filtered, and concentrated under reduced pressure. To the resultant crude crystals were added methanol (200 mL) and water (400 mL), which was heated at 100°C, and after confirming the dissolution of the crystals, the mixture was allowed to cool down to room temperature. Seed crystals (100 mg) were added to the mixture, followed by stirring at room temperature for 3 hours 40 minutes. The crystals were collected by filtration and dried to give 44.6 g of the title compound as white crystals.

¹H NMR (400 MHz, CDCl₃) δ: 1.35 (9H, s), 6.96 (1H, dt, J= 2, 8 Hz), 7.31 (1H, dt, *J* = 2, 8 Hz), 7.53 (1H, dd, *J* = 2, 8 Hz), 8.01 (1H, brs, No, 8.39 (1H, dd, *J* = 2,8 Hz).

### Example 3-B: N-[2-(1-Hydroxy-3,3,5,5-tetramethylcyclohexyl)phenyl]-2,2-dimethylpropionamide

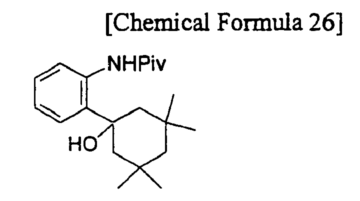

A solution of N-(2-bromophenyl)-2,2-dimethylpropionamide (3.9 g, 15.2 mmol) in tetrahydrofuran (39 mL) was cooled to -78°C under nitrogen atmosphere, and to this solution was added dropwise n-butyllithium (2.71 M solution in hexane, 14 mL, 38 mmol) at -78°C. The reaction mixture was stirred at the same temperature for 25 minutes, a solution of 3,3,5,5-tetramethylcyclohexanone (5.34 mL, 30,4 mmol) in tetrahydrofuran (8 mL) was added dropwise, followed by stirring at the same temperature for 1 hour 30 minutes. Water (10 mL) was added to the reaction mixture at -78°C, extraction was carried out with ethyl acetate. The organic layer was washed with a 5% aqueous solution of sodium chloride, dried over magnesium sulfate, filtered, and concentrated.
The reaction was carried out using N-(2-bromophenyl)-2,2-dimethylpropionamide (1 g, 3.73 mmol) by the same procedures as described above, and the resultant crude product was combined.
Methanol (100 mL) was added to the combined crude crystals, which was heated and stirred, and water (25 mL) was added while heating. After dissolution of the mixture, the mixture was allowed to cool down to room temperature and stirred at the same temperature for 2 hours. The crystals were collected by filtration and dried to give 4.1 g of the title compound as white crystals.

¹H NMR (400 MHz, CDCl₃) δ:0.96 (4.5H, s), 1.16-1.22 (1H, m), 1.33 (6H, s), 1.35 (4.5H, s), 1.46-1.52 (1H, m), 1.57 (6H, s), 1.60-1.71 (2H, m), 1.98-2.45 (2H, m), 7.02 (1H, dt, *J* = 2, 8 Hz), 7.18-7.36 (3H, m), 8.34 (1H, *J* = 8 Hz), 10.16 (1H, brs).

### Example 3-C: N-[2-(3.3.5.5-Tetramethylcyclohex-1-enyl)phenyl]-2.2-dimethylpropionamide

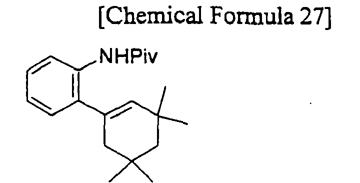

To a solution of N-[2-(1-hydroxy-3,3,5,5-tetramethylcyclohexyl)phenyl]-2,2-dimethylpropionamide (6.3 g, 19 mmol) in toluene (63mL) was added pyridinium p-toluenesulfonate (477 mg, 1.9 mmol) at room temperature, followed by stirring at 90°C for 2 hours. The reaction mixture was allowed to cool down to room temperature, and the organic layer was washed with water and a 5% aqueous solution of sodium chloride, dried over anhydrous magnesium sulfate, filtered, and concentrated to give 5.96 g of the title compound as a pale yellow solid.

¹H NMR (400 MHz, CDCl₃) δ:1.06 (6H, s), 1.12 (6H, s), 1.28 (9H, s), 1.46 (2H, s), 2.00 (2H, s), 5.52 (1H, s), 7.01-7.06 (2H, m), 7.20-7.24 (1H, m), 7.94 (1H, brs), 8.37 (1H, d, *J*= 8 Hz).

### ] Example 3-D: N-[2-(3,3,5,5-Tetramethylcyclohexyl)phenyl]-2,2-dimethylpropionamide

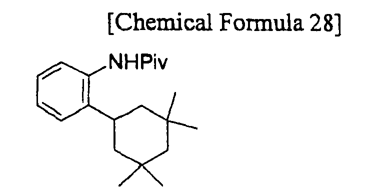

To a solution of N-[2-(3,3,5,5-Tetramethylcyclohex-1-enyl)phenyl]-2,2-dimethylpropionamide (5.7 g, 18.2 mmol) in ethanol (102 mL) was added 10% palladium on carbon (50% wet, 1.19g), followed by stirring at room temperature, at atmospheric pressure under hydrogen atmosphere for 3 hours. The reaction mixture was filtered through celite and concentrated to give 5.74 g of the title compound as a pale yellow solid.

¹H NMR (400 MHz, CDCl₃) 8:0.95 (6H, s), 1.12 (6H, s), 1.12-1.40 (4H, m), 1.35 (9H, s), 1.48-1.57 (2H, m), 2,99(1H, tt, J = 3, 12 Hz), 7.12-7.23 (3H, m), 7.24-7.30 (1H, m), 7.70 (1H, dd, *J* = 2, 8 Hz)

### Example 3-E: 2-(3,3,5,5-Tetramethylcyclohexyl)phenylamine

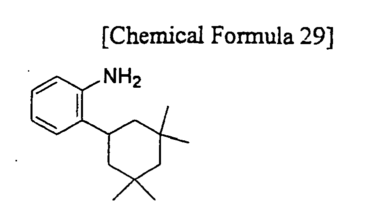

To a solution of N-[2-(3,3,5,5-Tetramethylcyclohexyl)phenyl]-2.2-dimethylpropionamide (330 mg, 1.05mmol) in ethylene glycol (6 mL) was added sodium methoxide (28%, 6.04 mL, 31.4 mmol) at room temperature, followed by stirring at 160°C for 8 hours and at room temperature for 15 hours 30 minutes. The reaction mixture was again stirred at 160°C for 5 hours. The reaction mixture was allowed to cool down to room temperature, diluted with t-butyl methyl ether, washed with water and a 5% aqueous solution of sodium chloride, dried over anhydrous magnesium sulfate, filtered, and concentrated to give 243 mg of the title compound as a brown oil.

### ] Example 4 :1-[2-(3,3,3,5,5-Tetramethylcyclohexyl)phenyl]piperazine (Reference)

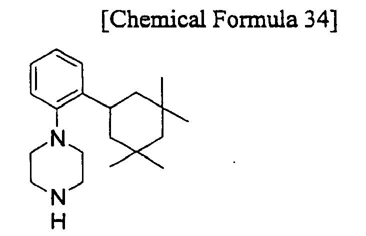

To a mixture of 2-(3,3,5,5-tetramethylcyclohexyl)phenylamine (168.0 g, 726.1 mmol) and 1,2-dichlorobenzene (1200 mL) was added bis(2-chloroethyl)amine hydrochloride (155.5 g, 871.3 mmol). The mixture was stirred for 7 hours at an external temperature of 190°C under a nitrogen atmosphere. During the reaction, a nitrogen stream was passed through the reactor several times to remove the generated hydrogen chloride gas. Reaction was repeated once more on the same scale as above, by the same procedure under the same reaction conditions. The two reaction mixtures were combined and subjected to the following treatment.

After cooling to room temperature, the combined reaction mixture was diluted with ethyl acetate (6 L) and water (1 L). The mixture was then added to a mixture of potassium carbonate (1.3 kg) and water (5 L) while stirring. The mixture was stirred and allowed to stand, and the organic layer was separated. The aqueous layer was again extracted with ethyl acetate (2 L). The combined organic layers were washed with brine (3 L) and then dried over anhydrous sodium sulfate (3.5 kg). The desiccant was removed by filtration and the filtrate was concentrated under reduced pressure. The resultant residue was purified by NH silica gel column chromatography (ethyl acetate/hexane) and then dried under reduced pressure to give 241.67 g of the title compound as a light pink solid.

In addition to this, the above NH silica gel column chromatography purification also yielded 126.2 g of an oil as a mixture of the target compound and impurities. Hexane (150 mL) was added to the oil, and the mixture was stirred for 2 hours at 0°C. The produced precipitate was collected by suction filtration and then dried under reduced pressure to give 42.74 g of the title compound as a light pink solid. A total of 284.41 g of the title compound was obtained as a light pink solid.

¹H-NMR (400MHz, CDCl₃)
δ : 0.93 (s, 6H), 1.13 (s, 6H), 1.17-1.35 (m, 4H), 1.42-1.46 (m, 2H), 2.84-2.87 (m, 4H), 3.02-3.04 (m, 4H), 3.60 (tt, J= 12.8, 2.8Hz, 1H), 7.06-7.18(m, 3H), 7.23 (dd, J= 7.6, 1.6Hz, 1H). The 1H ofNH could not be identified.

### Industrial Applicability

[2-(3,3,5,5-Tetramethylcyclohexyl)phenyl]piperazine compounds have excellent cell adhesion inhibitory action or cell infiltration inhibitory action, the invention can be useful in the production of the [2-(3,3,5,5-tetramethylcyclohexyl)phenyl]piperazine compounds.

## Claims

1. A process for producing compound (8a): 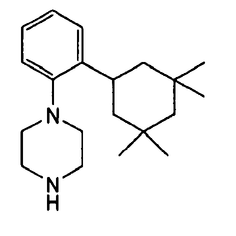 which comprises the reaction of compound (6a): 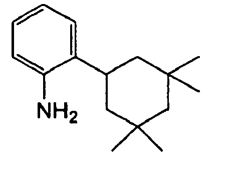 with compound (7a): 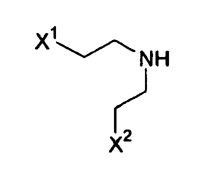 wherein X¹ and X² independently represent a leaving group
in a solvent, wherein the reaction is carried out in the presence of a base.

2. The process according to claim 1, wherein X¹ and X² are independently selected from halogen, methanesulfonyloxy and p-toluenesulfonyloxy.

3. The process according to claim 2, wherein X¹ and X² are independently selected from chlorine, bromine, iodine, methanesulfonyloxy and p-toluenesulfonyloxy.

4. The process according to claim 1, wherein the base is used in an amount of 1- to 10-fold molar with respect to the compound (6a).

5. The process according to claim 1, wherein the base is selected from potassium carbonate, sodium carbonate and triethylamine.

6. The process according to claim 1, wherein the compound (7a) is synthesized in situ by the reaction of diethanolamine with a halogenating agent or a reagent for introducing a leaving group.

7. The process according to claim 6, wherein the halogenating agent is selected from thionyl chloride, hydrochloric acid, phosphorus oxychloride, hydrobromic acid, N-bromosuccinimide and N-chlorosuccinimide, and the reagent for introducing a leaving group is selected from mesyl chloride and tosyl chloride.

8. The process according to claim 6, wherein the reaction of diethanolamine with the halogenating reagent or the reagent for introducing a leaving group is carried out in the presence of a base.

9. The process according to claim 1, wherein compound (7a) is used in an amount of 1- to 3-fold molar with respect to compound (6a).

10. The process according to claim 1, wherein the reaction of compound (6a) with compound (7a), or the reaction of diethanolamine with the halogenating agent or the reagent for introducing a leaving group is accelerated by combination with microwave.

## Patentansprüche

1. Verfahren zur Herstellung der Verbindung (8a): 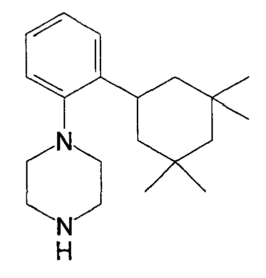 umfassend das Umsetzen der Verbindung (6a): 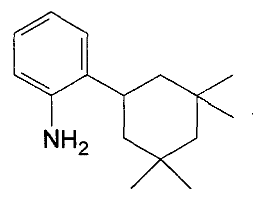 mit Verbindung (7a): 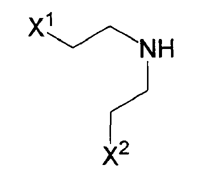 worin X¹ und X² unabhängig voneinander eine Abgangsgruppe darstellen,
in einem Lösungsmittel, wobei die Umsetzung in Gegenwart einer Base durchgeführt wird.

2. Verfahren gemäss Anspruch 1, wobei X¹ und X² unabhängig voneinander aus Halogen, Methansulfonyloxy und p-Toluolsulfonyloxy ausgewählt sind.

3. Verfahren gemäss Anspruch 2, wobei X¹ und X² unabhängig voneinander aus Chlor, Brom, Iod, Methansulfonyloxy und p-Toluolsulfonyloxy ausgewählt sind.

4. Verfahren gemäss Anspruch 1, wobei die Base in einer 1- bis 10-fachen molaren Menge, bezogen auf Verbindung (6a), verwendet wird.

5. Verfahren gemäss Anspruch 1, wobei die Base aus Kaliumcarbonat, Natriumcarbonat und Triethylamin ausgewählt ist.

6. Verfahren gemäss Anspruch 1, wobei die Verbindung (7a) durch Umsetzen von Diethanolamin mit einem Halogenierungsmittel oder einem Reagens zur Einführung einer Abgangsgruppe in situ synthetisiert wird.

7. Verfahren gemäss Anspruch 6, wobei das Halogenierungsmittel aus Thionylchlorid, Salzsäure, Phosphoroxychlorid, Bromwasserstoffsäure, N-Bromsuccinimid und N-Chlorsuccinimid ausgewählt ist und das Reagens zur Einführung einer Abgangsgruppe aus Mesylchlorid und Tosylchlorid ausgewählt ist.

8. Verfahren gemäss Anspruch 6, wobei die Umsetzung von Diethanolamin mit dem Halogenierungsmittel oder dem Reagens zur Einführung einer Abgangsgruppe in Gegenwart einer Base durchgeführt wird.

9. Verfahren gemäss Anspruch 1, wobei die Verbindung (7a) in einer 1- bis 3-fachen molaren Menge, bezogen auf die Verbindung (6a), verwendet wird.

10. Verfahren gemäss Anspruch 1, wobei die Umsetzung von Verbindung (6a) mit Verbindung (7a) oder die Umsetzung von Diethanolamin mit dem Halogenierungsmittel oder dem Reagens zur Einführung einer Abgangsgruppe in Kombination mit Mikrowellen beschleunigt wird.

## Revendications

1. Procédé de production d'un composé (8a) : 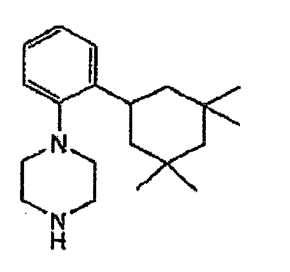 qui comprend la réaction d'un composé (6a) : 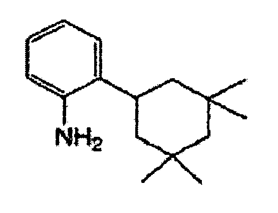 avec un composé (7a) : 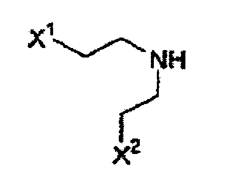 dans lequel X¹ et X² représentent indépendamment un groupe partant
dans un solvant, dans lequel la réaction est conduite en présence d'une base.

2. Procédé selon la revendication 1, dans lequel X¹ et X² sont indépendamment choisis parmi un halogène, un méthanesulfonyloxy et un p-toluènesulfonyloxy.

3. Procédé selon la revendication 2, dans lequel X¹ et X² sont indépendamment choisis parmi un chlore, un brome, un iode, un méthanesulfonyloxy et un p-toluènesulfonyloxy.

4. Procédé selon la revendication 1, dans lequel la base est utilisée dans une quantité de 1 à 10 fois molaire par rapport au composé (6a).

5. Procédé selon la revendication 1, dans lequel la base est choisie parmi le carbonate de potassium, le carbonate de sodium et la triéthylamine.

6. Procédé selon la revendication 1, dans lequel le composé (7a) est synthétisé *in situ* par la réaction de diéthanolamine avec un agent d'halogénation ou un réactif pour introduire un groupe partant.

7. Procédé selon la revendication 6, dans lequel l'agent d'halogénation est choisi parmi le chlorure de thionyle, l'acide chlorhydrique, l'oxychlorure de phosphore, l'acide bromhydrique, le N-bromosuccinimide et le N-chlorosuccinimide, et le réactif pour introduire un groupe partant est choisi parmi le chlorure de mésyle et le chlorure de tosyle.

8. Procédé selon la revendication 6, dans lequel la réaction de la diéthanolamine avec le réactif d'halogénation ou le réactif pour introduire un groupe partant est conduite en présence d'une base.

9. Procédé selon la revendication 1, dans lequel le composé (7a) est utilisé dans une quantité de 1 à 3 fois molaire par rapport au composé (6a).

10. Procédé selon la revendication 1, dans lequel la réaction du composé (6a) avec le composé (7a), ou la réaction de la diéthanolamine avec l'agent d'halogénation ou le réactif pour introduire un groupe partant est accélérée par combinaison avec une micro-onde.
